# EUROPEAN PATENT SPECIFICATION

(11) **EP 0 241 487 B1**
(45) Date of publication and mention of the grant of the patent: **22.04.1998**
(21) Application number: 86905604.4
(22) Date of filing: 28.08.1986
(51) Int. Cl.: C07H 21/00, C07H 1/08, C12P 19/30, C07K 5/00, C07K 7/00, C12N 15/00, C12P 21/00, G01N 33/53, G01N 33/68

(54) **METHOD AND MEANS FOR SORTING AND IDENTIFYING BIOLOGICAL INFORMATION**
VERFAHREN UND MITTEL ZUR SORTIERUNG UND BESTIMMUNG BIOLOGISCHER INFORMATIONEN
PROCEDE ET MOYEN DE TRIAGE ET D'IDENTIFICATION D'INFORMATIONS BIOLOGIQUES

(30) Priority: 28.08.1985 US 770390
(43) Date of publication of application: 21.10.1987
(73) Proprietor: PIECZENIK, George, New York, NY 10023 (US)
(72) Inventor: PIECZENIK, George, New York, NY 10023 (US)
(74) Representative: VOSSIUS & PARTNER
(86) International application number: US8601796
(87) International publication number: WO8701374

(56) References cited:
- EP-A- 0 048 470
- EP-A- 0 098 118
- EP-A- 0 135 277
- EP-A- 0 154 186
- EP-A- 0 157 643
- WO-A-84/02922
- WO-A-84/03564
- WO-A-85/00807
- WO-A-85/03725
- WO-A-86/00991
- DD-A- 143 794
- DE-A- 3 300 632
- NATURE, vol. 305, no. 5929, 01 September 1983, Chesham, Bucks (GB); G.N. GODSON et al., p. 29-33

## Description

This invention relates to the characterization and identification of the recognition sites of antibodies. More particularly, this invention involves the determination of the specific amino acid sequence recognized by an antibody, and of the nucleic acid sequence encoding that amino acid sequence.

The clonal selection theory of Burnet, which explains the general basis of antibody production, has gained virtually complete acceptance. Burnet, M. (1961) Sci. Am. 204 58; Jerne, N.K. (1976) Harvey Lecture 70 93. The theory is based on several premises: (1) as individual cells, i.e., lymphocytes, in the immune system differentiate, each becomes capable of producing only one species of antibody molecule; (2) the entire spectrum of possible antibody-producing cells is present within the lymphoid tissues prior to stimulation by any antigen; that is, the step in which each lymphocyte becomes specified to produce only one type of antibody molecule occurs in the absence of a potential antigen for that antibody; and (3) lymphocytes capable of producing an antibody specific to a particular antigen are induced, by the presence of that antigen, to proliferate and to produce large quantities of the antibody. An enormous range of genetically unique lymphoid cells is present in the lymphoid organs, e.g., the spleen, of each mammal. The spleen can be considered a library of cells, each of which can manufacture a unique antibody, and the library is so large that for any arbitrary antigen, at least one lymph cell exists within the library that is capable of recognizing the antigen and producing antibodies specific to the antigen.

Heretofore, the production of an antibody that will recognize an antigen of interest has required the antigenic stimulation of a laboratory animal. Typically, the antigen is injected into a laboratory animal, and, after a suitable incubation period, a second injection is given. The spleen cells of the animal are then harvested and fused to myeloma cells. When fused to a spleen cell, the myeloma cell confers to the spleen cell its ability to grow in culture. Surviving colonies of fused cells, i.e., hybridomas, are then screened to identify clones that produce antibodies that specifically recognize the antigen. This procedure must be repeated each time it is desired to produce an antibody to a particular antigen. For each antigen of interest, it is necessary to (1) antigenically stimulate an animal, (2) remove its spleen and hybridize the spleen cells with myeloma cells, and (3) dilute, culture, and screen clones for specific antibody production. Though antibodies that recognize the antigen are produced, this technique does not identify the epitope, i.e., the specific site on the antigen that an antibody recognizes; and one cannot direct the development of antibodies specific to a particular predetermined site or region of the antigen. Also, hybridoma techniques are not effective in the direct development of monoclonal antibodies that recognize haptens, i.e., molecules that contain antibody recognition sites, but which do not elicit an antigenic reaction when injected without a carrier into a laboratory animal. Since antigenic stimulation and antibody production are potentially hazardous to the host, the use of human hosts has been precluded in the development of monoclonal antibodies.

The binding domain of a monoclonal antibody specific to a malaria virus surface protein has been identified as being no larger than 40 amino acids long. Cochrane, A. H. et al, Proc. Natl. Acad. Sci. U.S.A. 79 5651 (1982), inserted a 340 base pair sequence from a Plasmodium knowlesi gene into the pBR 322 vector. The engineered vector produced in E. coli a beta-lactamase fusion polypeptide that reacted with a monoclonal antibody specific to a P. knowlesi circumsporozoite or CS protein. This finding indicated that the binding domain of the monoclonal antibody was limited to a region of the CS protein encoded by the inserted sequence, or approximately 110 amino acids. Lupski, J.R. et al., Science 220 1285 (1983), used the same system and, employing transposition mapping techniques, further localized the binding domain to a 40-amino acid region of the CS protein.

Green N. et al., published PCT application 84/00 687, produced antibodies by inoculating laboratory animals with synthetic peptides. Antibodies produced in response to peptides having a length of 8 to 40 amino acid residues and corresponding to sequences in an influenza virus protein were cross-reactive with the virus in vitro.

Dame, J.B. et al., Science 225 593 (1984), sequenced the CS gene of Plasmodium falciparum and discovered 41 tandem repeats of a tetrapeptide, with some minor variation. Using synthetic peptides of 4, 7, 11, and 15 amino acid residues of the predominant repeating amino acid sequence, Dame then conducted competitive binding assays to determine what length of peptide would inhibit the binding of the CS protein with a monoclonal antibody specific to that protein. Dame found that the synthetic 4 amino acid sequence did not significantly inhibit binding, but the 7, 11 and 15 amino acid sequences did inhibit binding. These results suggest that this monoclonal antibody to the CS protein recognizes a 5 to 7 amino acid sequence containing the repeating tetrapeptide.

WO84/03564 discloses a method for detecting the sequence of an epitope, of a given known protein, specific for a given antibody. The document teaches to produce a plurality of peptides, each of them comprising a sequence of a plurality of amino acids which corresponds to a sequence within the known amino acid sequence, and the said polypeptide having overlapping amino and sequences.

In one aspect the invention features a population of oligonucleotides, each containing between 1 and about 50 tandem sequences of the same length of from about 4 to about 12 nucleic acid triplets. Each oligonucleotide encodes for a corresponding oligopeptide of about 4 to about 12 L-amino acid residues, and the entire population represents at least about 10% of all oligopeptide sequences of the selected length. In preferred embodiments, each member of the oligonucleotide population has a single copy of the sequence of nucleotide triplets, the oligonucleotide sequence has between 5 and 7 triplets, and the oligonucleotide population is generated by random shearing of mammalian genetic material or is chemically synthesized from the component nucleic acids.

In a second aspect the invention features a process for the preparation of a population of oligopeptides coded for by the above-mentioned oligonucleotides containing between 1 and about 50 tandem sequences of the same length of about 4 to about 12 alpha-amino acid residues, and the population makes up at least 10% of all peptide sequences of the predetermined length. In preferred embodiments each member of the population has a single copy of the peptide sequence, the oligopeptide sequence has between 5 and 7 L-amino acid residues, and the population is generated by shearing of proteins or is chemically synthesized from the component L-amino acids.

In a third aspect, the invention features a population of vectors, wherein each member of the population comprises a substantially identical autonomously replicating nucleic acid sequence wherein at least a portion of each sequence is a structural gene, and an oligonucleotide insert as defined above recombinantly inserted into said structural gene within said vector, a significant proportion of the vector population being capable of expressing their recombinant structural genes when transferred into appropriate host cells, and wherein expression of the recombinant structural genes yields recombinant polypeptides comprising amino acid sequences as defined above. The invention also refers to the recombinant polypeptide population produced by the vector population as mentioned above, in particular to a population wherein each tandem unit of nucleotide triplets comprises from 5 to 7 nucleotide triplets.

In a fourth aspect, the invention features a matrix including a population of peptide sequences and a heterogeneous population of antibodies capable of binding to substantially all members of an oligopeptide population featured in the second aspect of the invention, above.

In a fifth aspect, the invention features a method for constructing the above matrix including the steps of (1) obtaining a population of polypeptides having a uniform length of between about 4 and about 12 alpha-amino acid residues and including at least about 10% of all peptide sequences of the predetermined length; (2) obtaining a heterogeneous population of antibodies capable of binding to substantially every member of the polypeptide population; and (3) contacting the antibodies with the antigens for a sufficient amount of time and under appropriate conditions so that binding occurs. In preferred embodiments: each of the peptide sequences and antibodies is isolated and each peptide sequence is contacted individually with each of the antibodies until at least one peptide sequence-antibody binding pair is identified; the peptide sequences can be immobilized on an appropriate substrate and the antibodies can be labeled; the antibodies can be immobilized and the peptide sequences can be labeled; or the peptide sequences can be excised from the polypeptides.

The invention provides an efficient and convenient means for the production of monoclonal antibodies to any specific region of any antigen or hapten of interest. Monoclonal antibody production, according to the invention, does not require antigenic stimulation of a host animal. The invention involves the antibody binding properties of a test species, but is totally independent of the ability of the test species to induce an antigenic response in vivo. The invention permits the identification of the specific peptide sequence on a protein that is recognized by an antibody. The specificity of antibodies recognizing distinct sequences on the same antigen can be differentiated. In addition, the invention permits the characterization and the localization on a chromosome of the nucleotide sequence encoding for the amino acid sequence recognized by an antibody.

Using conventional monoclonal techniques, one can produce antibodies that might react, for example, with an undetermined site on a particular Plasmodium circumsporozoite protein or a particular influenza virus. Using the present invention, one can identify all the epitopes on that molecule or organism and obtain an antibody that recognizes each of these epitopes. An epitope is a specific site on the surface of an antigen that is recognized by an antibody. By judiciously combining a number of distinct antibodies, each of which recognizes a different epitope on the surface of a particular antigen, a material with any desired degree of specificity can be obtained. Also using the invention, one can identify sequences that are common to, e.g., the circumsporozoite proteins of several Plasmodium species or to several strains of influenza, and screen for antibodies recognizing these cbmmon sequences, thereby identifying a single set of antibodies, each of which is effective against a broad range of malarial or influenza infections.

Certain viruses, such as the LAV or HTLV-III virus, contain on their surfaces both highly mutable regions and constant regions. The viruses' ability to alter their surface characteristics has hampered the development, through standard monoclonal techniques, of antibodies to these viruses. Any antibody that recognizes a mutable region of a virus would become ineffective as the virus mutated and a strain developed having an altered configuration in the region recognized by the antibody. Once the constant regions of a virus have been identified and characterized, the invention permits the identification and production of antibodies that recognize these constant regions, even if the peptide sequences comprising these constant regions would not themselves elicit an immunogenic response in vivo. Such antibodies would be effective against various strains of the virus.

Other features and advantages of the invention will be apparent from the following description of the preferred embodiments and from the claims.

### Preferred Embodiments

It is believed that an epitope has limited dimensions of between about 30 and 50 angstroms. An antibody that recognizes a specific peptide sequence or configuration of carbohydrates on the surface of an antigen will recognize that same configuration if it is duplicated or closely approximated on a different antigen. This phenomenon underlies the cross-reactivity sometimes encountered with monoclonal antibodies.

The size of the antibody recognition site corresponds to a peptide sequence in the range of between about 4 and about 12 amino acid residues. Mammalian proteins and polypeptides are composed almost exclusively of the twenty naturally occurring amino acids, i.e., glycine and the L-isomers of alanine, valine, leucine, isoleucine, proline, phenylalanine, tyrosine, tryptophan, serine, threonine, aspartic acid, glutamic acid, asparagine, glutamine, cysteine, methionine, histidine, lysine, and arginine. There are about three million (20⁵) different possible sequences of the twenty amino acid residues taken five at a time, and about sixty million if the amino acid residues are taken six at a time. This finite number of peptide sequences may represent the full range of possible antibody recognition sites. Production and maintenance of a representative sample of the full range of antibodies and of a representative sample of the peptide sequences of the appropriate length provides the means (1) to screen any antibody of interest in order to determine the precise peptide sequence it binds to and (2) to screen any protein in order to find an antibody specific to that protein.

The present invention identifies antibody binding sites that comprise a primary peptide sequence or, e.g., a carbohydrate sequence that is closely approximated by a peptide sequence.

Notwithstanding these beliefs, the invention provides the means and methods for the identification and characterization of epitopes, and of the antibodies that bind to them.

### Antibody production

According to the clonal selection theory, an unchallenged mammalian host has the capacity to produce antibodies to a vast array of foreign antigens. The presence of an antigen triggers the proliferation of those lymphocytes already present having the ability to produce antibodies to the antigen. Since there is a finite number of peptide sequences of the length that is recognized by antibodies, it can be expected that each mammal has the capability to produce antibodies that will recognize most if not all of these sequences. Thus the spleen of a mouse or another laboratory animal can serve as an appropriate source for a full range of antibodies. The spleen can be harvested from a laboratory animal, and, using standard techniques, the individual cells are fused to myeloma cells and hybridoma strains are developed.

Depending on the desired characteristics of the resulting hybridoma population, either antigenically stimulated animals can be used, or animals that have not been specifically challenged with the antigenic material of interest can be used.

If antigenically stimulated animals are used, then a higher proportion of the resulting hybridomas will produce antibodies specific to the antigen used. If, on the other hand, unchallenged animals are used, then it can be expected that the antibodies retrieved from the resulting population of hybridomas will represent a broader range of the antibodies that the animals are capable of producing. The antibodies produced by a mature animal raised under standard laboratory conditions will reflect and be limited by its individual exposure history. If spleens are harvested from several unchallenged mature animals and combined together, and the spleen cells fused to myeloma cells, then the resulting population of hybridomas will produce a more complete range of antibodies then would hybridomas from any single individual. Antibodies produced by the hybridomas derived from the spleen cells of mature animals that were raised aseptically or from fetal or neonatal animals will not reflect any exposure history and can be expected to represent a random sample of the full range of antibodies that the animals are capable of producing.

Since this procedure does not require antigenic stimulation of the donor animal before harvesting the spleen, it is now possible to develop antibodies derived from human cells. Normal spleen cells can be collected from one or a number of human donors and the harvested cells fused to myeloma cells and cultured as described above. Alternatively, a library of human antibodies can be developed over time by obtaining cell cultures from, e.g., a large number of myeloma patients, each patient having a distinctive tumor.

### Production of peptide sequences

The production of the desired population of peptides coded for by the oligonucleotides as defined above comprises producing them either by randomly shearing proteins and then recovering by electrophoresis the peptide sequences of the appropriate length, or by synthesizing the desired peptide sequences from their component amino acids.

Alternatively, these peptides can be produced through genetic engineering techniques. Peptides produced according to this general method can be termed coded peptides. A population of nucleotide sequences is first obtained of the correct length to encode for peptide sequences of the desired length. This can be accomplished either by random cleavage of biological genetic material followed by electrophoresis to recover those nucleotide sequences that were sheared to the desired length, or by synthesis from the component nucleic acids.

Depending on the desired characteristics of the resulting population of nucleotide sequences and ultimately, of the peptide sequences to be produced, different techniques are used to obtain the population of nucleotides. If a random population of nucleotide sequences is desired, then the nucleotides can be syntnesized by adding the four nucleic acids with equal frequency at each position of the growing nucleotide chains. If it is desired that the synthesized nucleotide triplets more closely reflect the distribution of naturally occurring triplets, then the frequency of each nucleic acid employed at the first, second, or third position of each triplet can be manipulated to approximate the frequencies at which each nucleic acid residue appears at each position in nature, as suggested in Crick F.H.C. et al., Origin of Life, 7 389-397 (1976). Any of several sources of genetic material can be selected to obtain by shearing nucleotide sequences of the desired length, e.g., cellular DNA or cDNA. cDNA, of course, would provide a tighter representation of the naturally occurring coding sequences.

When the desired population of nucleotide sequences has been obtained, the population can then be treated to facilitate the insertion of each sequence into a vector and to facilitate the subsequent recovery of the desired peptide sequence from the culture of host cells incorporating the engineered vector. For example, using known techniques, AUG sequences can be ligated to each end of each member of the population of nucleotide sequences. When each nucleotide sequence is translated, the desired peptide sequence will be flanked by methionine residues. The translated protein can then be treated with cyanogen bromide, which cleaves peptides at methionine sites, to excise the desired peptide sequence from the protein. The cleavage product can then be purified by electrophoresis. Alternatively, a restriction endonuclease recognition sequence can be ligated to each end of each member of the population of nucleotide sequences and then the population of nucleotide sequences can be treated with the endonuclease recognizing the ligated sequence to produce "sticky ends" which facilitate the insertion of the nucleotide at the restriction site in a vector recognized by the endonuclease.

Each nucleotide sequence is then inserted into an appropriate vector. The ratio of nucleotide sequences to vectors can be controlled to ensure that no more than one nucleotide sequence is inserted into any vector. The nucleotide sequence must be inserted at a location in the vector where it will be translated in phase when the vector is transferred into an appropriate host cell, and where it will not interfere with the replication of the vector under the experimental conditions employed. The nucleotide sequence must be inserted into a non-essential region of the vector. Pieczenik, U.S. Patent 4,359,535, discloses a method for inserting foreign DNA into a non-essential region of a vector.

The nucleotide sequence is advantageously inserted in such a way that the peptide sequence encoded by the nucleotide sequence is expressed on the outside surface of the vector. To prepare inserts having these characteristics, an appropriate vector, e.g., a phage or plasmid, is first selected. The vector is then randomly cleaved according to the method disclosed in Pieczenik, U.S. Patent 4,359,535, to yield a population of linear DNA molecules having circularly permuted sequences. After the cleavage steps, a synthetic oligonucleotide linker bearing a unique nucleotide sequence not present on the original unmodified vector can be attached to both ends of each linearized vector by blunt end ligation. The random linears can then be treated with the restriction endonuclease specific to the attached sequences, to generate cohesive ends.

DNA encoding a gene product, e.g., human hemoglobin, not present in the vector, is fractionated to the desired size, e.g., fifteen nucleotides long, and the nucleotide sequences ligated to the same type of linker used with the random linears. The fractionated nucleotide sequences are then inserted into the random linears, and the modified vectors are transferred into appropriate host cells. The host cells are diluted, plated, and the individual colonies grown up. On replica plates, the colonies are screened with a monoclonal or polyclonal antibody specific to the gene product.

A positive reaction with the antibody identifies a colony wherein the inserted nucleotide sequence is translated in phase, and the encoded peptide sequence is on the outside surface of the polypeptide or protein, accessible to the antibody screening assay. If a monoclonal antibody is employed in the screening step, then this procedure will identify only those colonies where the specific peptide sequence comprising the site recognized by that antibody is inserted on the outside surface of the polypeptide or protein. If a polyclonal antibody is employed, or a mixture of several monoclonals, then any colony containing on the outside surface of the polypeptide or protein any peptide sequence insert comprising a recognition site of the foreign gene product will be identified. This procedure identifies vectors which can be advantageously used in the present invention.

The insertion step creates a population of vectors, each containing a nucleotide insert encoding for a different peptide sequence, each encoded peptide sequence containing the same desired number of amino acid residues. This population of vectors is then transferred into a population of appropriate host cells. Concentrations of vectors and of host cells can be controlled to ensure that no more than one vector is transferred into any host cell. Cells are plated and cultured, and the translated proteins are harvested therefrom.

### Creating the Matrix

The particular construction of the matrix created from the full range of antibodies or from the peptide sequences described above depends on its use. Either the antibodies or the peptide sequences are immobilized on a substrate, e.g., nitrocellulose. The immobilization can be accomplished by covalently linking the antibodies or peptide sequences to the substrate. Each site on the matrix is occupied by a single chemical species, i.e., a monoclonal antibody or a purified peptide. The source of each individual immobilized species is maintained as a separate culture. In general, the antibodies peptide sequences, or the test species are labeled with an appropriate label, such as a fluorescent compound, an enzyme, or a radioactive tracer. The peptide sequence itself can serve as a sensitive biological tag where it occurs on the surface of a protein or vector.

Where the antibodies are immobilized, the peptide sequences are then contacted with the antibodies under appropriate conditions and for a sufficient amount of time so that each immobilized antibody binds to the peptide sequence to which it is specific. Where the peptide sequences are immobilized, the antibodies are then contacted with the peptide sequences so that each immobilized peptide sequence is recognized and bound by an antibody specific to that sequence. Each complex of peptide sequence and its bound antibody can be termed a binding pair. In some cases, the antibodies or peptide sequences themselves are immobilized on the substrate; in other cases the cell cultures producing the antibodies or peptides are immobilized. Binding pairs are created in a single step, taking advantage of the natural affinity of antibodies for the peptide sequences to which they are specific. If a sample of peptides is contacted with a population of immobilized antibodies, then the peptides will self-sort and each will bind to its corresponding antibody. Similarly, if a sample of antibodies is contacted with a population of immobilized peptides, then the antibodies will self-sort and each will bind to its corresponding peptide. The sorting will occur notwithstanding that there is no prior knowledge as to the functional characteristics of any of the individual antibodies or peptides.

A matrix where the antibodies are immobilized on the substrate will be designated an antibody-immobilized matrix, or AIM. Where each immobilized antibody forms a binding pair with a corresponding peptide sequence, the matrix will be designated P-AIM. Similarly, a matrix where the peptide sequences are immobilized on the substrate will be designated a peptide-immobilized matrix, or PIM. Where each immobilized peptide sequence forms a binding pair with a corresponding antibody, the matrix will be designated A-PIM.

Generally, the method of the invention involves contacting a test species with an intact P-AIM or an intact A-PIM, the specific characteristics of the matrix depending on the nature of the information sought. Considering the large number of different hybridomas and genetically engineered clones that are involved in the procedure of the invention, the antibodies or peptide sequences can be immobilized very densely on the substrate. Areas of competitive binding are identified when the test species is contacted with the matrix. Colonies from these areas can then be retrieved, replated less densely, and the competitive binding step with the test species repeated in order to specifically identify the individual colony producing the antibody or amino acid sequence where pairing was disturbed.

### Screening an Antibody or Test Species of Interest

A P-AIM is used both to identify and obtain antibody clones that are specific to a test species of interest and to identify the specific peptide sequence recognized by an antibody of interest. The test species can be, for example, a virus, a bacteriophage, a virus coat protein, a surface protein of a viral or bacterial pathogen, a protein on the surface of a malignant cell, an enzyme, or a peptide having the sequence of a selected portion of a protein of interest. The test species need not contain peptides, but may be, e.g., a drug or carbohydrate having a configuration that is closely approximated by a peptide sequence.

The test species is contacted with a P-AIM in a competitive binding assay with each of the complexed binding pairs. Each binding pair occupies a unique site on the matrix.

Where these pairs have been labeled, any pairings disturbed by the presence of the test species can be identified.

A particularly sensitive labeling technique is obtained where the peptide sequences bound to the immobilized antibodies are on the surface of a protein or vector. After the P-AIM is created and the binding pairs are established, the P-AIM is thoroughly washed to remove any unbound peptide sequences. The test species is then contacted with the P-AIM. Any peptide sequences that are displaced from their corresponding antibodies by the presence of the test species can be directly titered off the P-AIM. Available techniques are sufficiently sensitive to detect the presence of as few as ten molecules of protein or vector organisms in the titered supernatant.

Where the test species is labeled, its binding can be detected directly. Each clone producing an antibody that binds to a test species is identified and cultured to provide a source of the antibody. Each culture producing a peptide sequence displaced by the presence of an antibody of interest is identified and cultured to provide a source of that peptide sequence.

A PIM is used both to identify the specific sequences on a test protein or polypeptide that can be recognized by antibodies and to identify the specific peptide sequences recognized by an antibody of interest. The procedure for screening on a PIM is analogous to the procedure, above, for screening on an AIM. The test protein or peptide sequence, or the test antibody, is contacted with an intact A-PIM in a competitive binding assay with each of the antibody-peptide sequence pairs. The pairings disturbed by the presence of the test protein or polypeptide or test antibody are noted, and the clones producing the amino acid sequence to which pairing was disturbed are identified and cultured. By this method, not only is it possible to determine the amino acid sequence recognized by the antibody, but it is now possible as well to identify the nucleic acid sequence encoding this amino acid sequence, as the insert in the vector contained in the clone that produces the recognized amino acid sequence.

### Example I

To illustrate certain aspects of the present invention, a method for determining the antibody recognition sites on insulin will now be described.

### Production of hybridoma cell lines.

Several C57B1/10 mice are immunized intraperitoneally with 100 micrograms of human insulin precipitated in alum, mixed with 2x10⁹ killed Bordetella pertussis organisms as adjuvent. A second injection of 100-200 micrograms of insulin in saline is given a month later.

Three days after the second injection, the mice are killed by neck dislocation, the spleens are removed aseptically and transferred into a bacteriological-type plastic petri dish containing 10 ml of GKN solution. GKN solution contains, per 1 liter of distilled water: 8 g NaCl, 0.4 g KCl, 1.77 g Na₂HPO₄^{·}2H₂O, 0.69 g NaH₂PO₄^{·}H₂O, 2 g glucose, and 0.01 g phenol red. The cells are teased from the capsule with a spatula. Clumps of cells are further dispersed by pipetting up and down with a 10 ml plastic pipette. The suspension is transferred to a 15 ml polypropylene tube where clumps are allowed to settle for 2 to 3 minutes. The cell suspension is decanted into another tube and centrifuged for 15 minutes at 170 G at room temperature. The cells are washed again in GKN and finally resuspended in 1-2 ml GKN. A 20 microliter aliquot of the cell suspension, stained with 1 ml of trypan blue solution, is counted to determine the yield of spleen cells.

10⁸ washed spleen cells and 5 x 10⁷ 8-azaguanine resistant myeloma cells (e.g., cell line X63Ag8.6.5.3; FO; or Sp2/0-Ag14) are combined in a 50 ml conical tube (Falcon 2070). The tube is filled with GKN and spun at 170-200:G at room temperature. The supernatant is then withdrawn, and 0.5 ml of a 50% solution of polyethylene glycol in GKN is added dropwise to the pellet. This addition is accomplished over a one minute period at room temperature as the pellet is broken up by agitation. After 90 seconds 5-10 ml of GKN are added slowly over a period of 5 minutes. The cell suspension is then left for 10 minutes, after which large clumps of cells are dispersed by gentle pipetting with a 10 ml pipette. The cell suspension is then diluted into 500 ml of Dulbecco's modified Eagles medium containing 10% fetal calf serum and HAT. 1 ml aliquots are distributed into 480 wells of Costar-Trays (Costar Tissue Culture Cluster 24, Cat. No. 3524, Costar, 205 Broadway, Cambridge, MA) already containing 1 ml HAT medium and 10⁵ peritoneal cells or 10⁶ spleen cells. The trays are kept in a fully humidified incubator at 37°C in an atmosphere of 5% CO₂ in air. After 3 days and twice a week thereafter, 1 ml medium is removed from each well and replaced with HAT medium. After 7-10 days the wells are inspected for hybrids and the HAT medium is replaced with HT medium. Cell populations of interest are expanded by transfer into cell culture bottles for freezing, cloning, and product analysis. 10⁶ peritoneal cells are added at this time to each culture bottle.

Hybridomas produced by the methods outlined above are propagated and cloned, using standard techniques. The monoclonal antibody produced by each hybridoma line is purified from the culture supernatant and concentrated by affinity chromatography on a protein A-sepharose column.

### Production of gene library

cDNA is synthesized from a heterogeneous population of mRNA. The cDNA is randomly sheared and the 15 nucleotide fragments are retrieved by electrophoresis. These fragments are inserted, in phase, into the structural gene encoding beta-galactosidase of lambda-gt 11, according to the method disclosed in Pieczenik, U.S. Patent 4,359,535. Each of the resulting clones produces the normal lambda-gt 11 protein containing a foreign sequence of 5 amino acid residues encoded by the 15 nucleotide fragment inserted into the beta-galactosidase gene.

### Screening and precise identification of the antibody binding sites

The library is plated at a density of 25,000 plaques per 150-mm² plate and immunologically screened, using a pool of those monoclonal antibodies reactive with human insulin and unreactive with unmodified lambda-gt 11 phage. The immunological screening is carried out essentially according to the method described by Young, R.A. et al. Science (1983) 222, 778, which is hereby incorporated by reference.

The lambda-gt 11 clones identified by the screening procedure are introduced as lysogens into E. coli strain RY 1089 (ATCC 37,196). Lysogens are grown at 32°C in media containing 50 micrograms of ampicillin per milliliter until absorbance at 550mm is 0.4 to 0.8. The phages are induced at 44°C by shaking gently for 20 minutes and then isopropyl-thiogalactoside (IPTG) is added to a final concentration of 2mM, and the culture is shaken an additional hour at 37°C in order to enhance expression of beta-galactosidase and possible fusion proteins.

Lysates are then subjected to electrophoresis on a sodium dodecyl sulfate-polyacrylamide gel (SDS-PAGE) and electroblotted into nitrocellulose. Pelleted cells from 0.1 ml of each lysogen culture are dissolved in 20 microliters of SDS gel sample buffer (3% SDS, 10% glycerol, 10 mM dithiothreitol, 62 mM tris-HCl, pH 6.8) at 95°C for 5 minutes for electrophoresis. Western blot analysis is performed according to a modification of the method of Towbin H. et al. (1979) Proc. Natl. Acad. Sci. U.S.A. 79 4350. Proteins are separated by SDS-PAGE according to the method of Laemmli (1970) Nature 277 680 with a 4.5% stacking gel and an 8-12% gradient gel. The filter is reacted for 90 minutes with a single one of the monoclonal antibodies selected above diluted to a concentration of 1:20,000 with PBS containing 0.05% Tween-20 and 20% FCS. Filter-bound antibody is incubated with ¹²⁵I-labeled sheep antiserum prepared against whole mouse antibody (diluted to 2 X 10⁵ cpm/ml with PBS containing 0.05% Tween-20 and 20% FCS) and then detected by autoradiography. The lysogen that is reactive with the specific antibody used contains the engineered lambda-gt 11 clone whose beta-galactosidase enzyme is fused to a 5 amino acid sequence that corresponds to the 5 amino acid sequence of insulin recognized by the antibody. The electrophoresis and electroblotting steps are repeated for each of the monoclonal antibodies selected above, and the specific sequences on the insulin molecule recognized by each of these antibodies is identified.

### Example II

The method of Example 1 is modified to eliminate the step of inoculating the mice with human insulin. An identical harvesting procedure is used to obtain spleen cells from mice that have not been antigenically stimulated. The spleen cells are hybridized with myeloma cells as described in Example 1, and the resulting hybridomas are propagated and cloned. Notwithstanding the elimination of the antigenic stimulation step, screening identifies clones that produce antibodies reactive with human insulin.

### Example III

To further illustrate the invention, a method for creating and screening a cDNA expression library will now be described. In this example, the cDNA library is prepared from chicken smooth muscle mRNA.

### Production of Gene Library

Total smooth muscle RNA is prepared from 11-day embryonic chicken stomachs and gizzards according to the method of Chirgwin, J.M. et al., (1979) Biochemistry 18 5294 and Feramisco, J.R. et al., (1982) J. Biol. Chem. 257 11024. Poly (A)+ RNA is isolated by two cycles of adsorption to and elution from oligo(dT)-cellulose according to the method of Aviv, H. et al., (1972) Proc. Natl. Acad. Sci. USA 69 1408. Starting with about 25 micrograms of poly(A) +RNA, first and second strand cDNA is synthesized using avian myeloblastosis virus reverse transcriptase. The double linker method of Kartz and Nicodemus, (1981) Gene 13 145 can be employed. The double stranded cDNA, with intact hairpin loops at the ends corresponding to the 5' ends of the poly(A)+ mRNA, are filled in with the Klenow fragment of E. coli DNA polymerase I (available from Boehringer Mannheim or New England BioLabs). The filled in cDNA is then ligated to ³²P-labeled Sal I octanucleotide linkers (available from Collaborative Research, Waltham MA). The cDNA with Sal I linkers attached to the end corresponding to the 3' end of the poly(A)+ mRNA is then treated with nuclease S1 to destroy the hairpin loop and again is filled in with the Klenow fragment of E. coli DNA polymerase I. EcoRI octanucleotide linkers (also available from Collaborative Research) are ligated to the cDNA. The DNA is digested to completion with both EcoRI and Sal I. A Sepharose 4B column equilibrated with 10mM Tris-HCl (pH 7.6) containing 1 mM EDTA and 300 mM NaCl is used to isolate and purify those cDNA fragments 15 nucleotides long flanked by the two octanucleotide linkers.

The plasmid vector pUC8, described in Vieria et al. Gene 19 (1982) 259, is digested to completion with EcoRI and Sal I and extracted twice with a 1:1 by volume mixture of phenol and chloroform. The 2.9 kilobase fragment is separated from the 16 nucleotide long fragment on a Sepharose 413 column, equilibrated as set forth above. Fractions containing the large fragment are-pooled and precipitated with ethanol. cDNA is ligated to the vector at a weight ratio of vector to cDNA of 1000:1. Approximately 1 nanogram of cDNA is ligated to 1 microgram of the plasmid vector.

Conventional techniques are employed to transform E. coli strain DH-1 with the engineered pUC8 vector. The bacteria are plated onto 82 mm nitrocellulose filters (Millipore Triton-free HATF) overlaid on amplicillin plates to give about 1,000 colonies per filter. Colonies are replica plated onto nitrocellulose sheets (available from Schleicher & Schüell) and the replicas are regrown both on selective plates for antibody and hybridization screening and on glycerol plates for long-term storage at -70°C.

### Antibody Production and Immunological Screening

Each plate is immunologically screened to identify colonies where the plasmid contains a 15 nucleotide cDNA insert corresponding to a portion of the chicken tropomyosin gene. Monoclonal antibodies for use in the screening are developed as follows.

Spleen cells are harvested from donor mice that have been antigenically stimulated with chicken tropomyosin. Alternatively, spleen cells are harvested from mice that have not been antigenically stimulated. The spleen cells are fused to myeloma cells to produce hybridoma strains. The monoclonal antibody produced by each hybridoma line is purified from the culture supernatant and concentrated by affinity chromatography on a protein A sepharose column.

Antibodies are screened for reactivity with chicken tropomyosin and with the parental bacterial strain, DH-1, which does not contain a plasmid. Those antibodies reactive with the tropomyosin and unreactive with DH-1 are selected for use in screening the transformed bacterial colonies.

To prepare the bacterial colonies for screening, they are lysed by suspending the nitrocellulose filters for fifteen minutes in an atmosphere saturated with CHCl₃ vapor. Each filter is then placed in an individual Petri dish in 10 ml of 50 mM Tris-HCl, pH 7.5/150 mM NaCl/5 mM MgCl₂ containing 3% (wt/vol) bovine serum albumin, 1 microgram of DNase, and 40 micrograms of lysozyme per milliliter. Each filter is agitated gently overnight at room temperature, and then rinsed in saline (50 mM Tris-HCl, pH 7.5/150 mM NaCl). Each filter is incubated with a dilute saline solution of a monoclonal antibody selected from those antibodies exhibiting reactivity with tropomyosin but not with DH-1. The filters then are washed five times with saline at room temperature, from one half to one hour per wash. The filters then are incubated with 5 x 10⁶ cpm of ¹²⁵I-labeled goat anti-mouse IgG having a specific activity of about 10⁷ cpm/microgram and diluted in 10 ml of saline containing 3% bovine serum albumin. The goat anti-mouse IgG can be an affinity purified fraction. The labeling is accomplished according to the chloramine-T procedure of Burridge, K. (1978) Methods Enzymol. 50 57. After one hour of incubation the filters are washed again in saline, with five or six changes, at room temperature, dried, and autoradiographed 24-72 hours using Dupont Cronex Lightning Plus x-ray enhancing screens. In the immunological screenings, a filter is advantageously included upon which defined amounts of various purified proteins are spotted. This serves as a further control for the specificity of the immunological detection of the antigens. Quantities of less than 1 nanogram of purified protein can be detected in these assays.

This procedure permits the identification and characterization of the specific five peptide sequence of the tropomyosin protein that is identified by a particular monoclonal antibody. As this immunological screening process is repeated with different monoclonal antibodies, several distinct antigenic sites on the tropomyosin protein are identified. The 15 nucleotide sequence of cDNA that encodes for each antigenic site is preserved in the cDNA library, and a source of antibody that recognizes each site is preserved in the separate hybridoma lines.

### Use

The invention is useful to produce antibodies that recognize and bind to particular test species, and to determine either (1) the specific peptide sequence on a protein, enzyme, or peptide that an antibody recognizes or (2) an amino acid sequence with a configuration very close to the structure of a non-peptide test species recognized by an antibody. The invention is also useful to determine the nucleotide sequence encoding the amino acid sequence that is recognized by an antibody.

To identify a peptide sequence that closely approximates an antibody binding site on a test species, either an A-PIM or a P-AIM can be used. If an A-PIM is used, then the test species is first contacted with the intact A-PIM. Any antibodies bound to immobilized peptide sequences that have an affinity for the test species will be "competed off" the matrix to bind to the test species. The peptide sequence immobilized at a site where antibodies are "competed off" has a conformational similarity to the site on the test species where the antibodies are now bound. If a P-AIM is used, then the test species is first contacted with the intact P-AIM. The test species displaces any peptide sequences that have a sufficient conformational similarity to an antibody recognition site on the test species that an antibody capable of binding to the peptide sequence is also capable of binding to the test species. Displaced peptide sequences can then be titered off the matrix and identified. It is not necessary that the test species be proteinaceous or derived from peptides. It can be, for example, a carbohydrate or a non-peptide drug. It can be expected that the recognition site of a non-peptide substance is closely approximated by the conformation of a peptide sequence. A test species can disturb the binding at more than a single site on a matrix; this could occur because there is more than one distinct antibody recognition site on the test species or because two or more distinct peptide sequences are each similar in conformation to a recognition site on the test species. It is not necessary that the test species be immunogenic, i.e., induce the production of antibodies in vivo if innoculated into a mammal; the antibody binding sites of a test species can be characterized notwithstanding that the test species is not immunogenic.

Where the test species is a disease producing agent, such as a virus or bacteria, then the peptide sequences that are similar in conformation to the antibody recognition sites of the disease producing agent can be employed to develop a vaccine. A synthetic antigen incorporating the identified peptide sequence or sequences, when injected into a patient's bloodstream, induces the production of antibodies against the disease producing agent.

Where the test species is the recombinant gene product of a gene expression library, one is able to determine precisely what regions of the gene product make up antibody recognition sites. The identified peptide sequences correspond to sequences contained in the gene product that are recognized by antibodies.

Where the test species is a gene product, such as, for example, a protein, enzyme, or peptide, then the invention also provides a means for locating in a genome the gene encoding for the gene product. After the peptide sequences identified from screening the gene product through the matrix are identified, the recombinant cell lines that produced those peptide sequences are identified and the recombinant nucleotide sequences encoding those peptide sequences are recovered. The nucleotide sequences can then be used as a DNA probe to locate on the genome the gene encoding for the gene product. Since each nucleotide sequence is fairly short, i.e., from 5 to 12 triplets in length, it can be expected that any one sequence, or a closely similar sequence, would be repeated more than once in the genome. Therefore, several distinct nucleotide sequences, each encoding a distinct peptide sequence, are advantageously employed in a DNA probe. A region on a chromosome where several nucleotide sequences hybridize in close proximity identifies the gene encoding for the gene product.

To determine the peptide sequence recognized by a particular antibody of interest, either a PIM or a P-AIM can be used. If a PIM is used, it is not necessary that each immobilized peptide sequence be bound to a corresponding antibody. The antibody of interest can be contacted directly with a matrix of immobilized peptide sequences. Any immobilized sequences that are bound by the antibody of interest can then be directly identified. If a P-AIM is used, then the antibody of interest is first contacted with the intact P-AIM. Any peptide sequences bound to immobilized antibodies that can be recognized by the antibody of interest will be "competed off" the matrix to bind with the antibody of interest. Peptide sequences that have been "competed off" the matrix by the presence of the antibody of interest can then be titered off the matrix and identified.

Where it is desired to determine the nucleotide sequence encoding the peptide sequence recognized by an antibody of interest, the recombinant cell line that produces the peptide sequence recognized by the antibody can be identified and the nucleotide sequence encoding the peptide sequence can be recovered and sequenced.

Where the antibody of interest is an antibody produced by a patient suffering from an autoimmune disease and the antibody attacks the patient's own cells, impairing the functioning of those cells, then the peptide sequence recognized by the antibody can provide a basis for treating the patient. The peptide sequence recognized by the antibody can be administered to the patient in an effective amount to competitively inhibit the antibody from attacking the patient's own cells in vivo. The patient's condition will be improved since fewer antibodies will be available to attack the living cells, and the administration of peptides will not induce further antibody production since the peptides are too short to induce an immunogenic response.

To identify an antibody that reacts with a test species, an AIM is used. It is not necessary that each immobilized antibody be bound to a corresponding peptide sequence. The test species can be contacted directly with a matrix of immobilized antibodies. Any immobilized antibodies bound to the test species can be directly identified, and the clones producing those antibodies can be cultured to provide a source of the antibodies. It is not necessary that the test species be proteinaceous or derived from peptides. It can be, for example, a carbohydrate or a non-peptide drug. It is not necessary that the test species be immunogenic. It is possible to obtain antibodies that recognize a test species notwithstanding that the test species does not induce antibody production in vivo.

The antibodies that recognize the test species can be used in an immunoassay to test for the presence of the test species in a biological sample.

Where the test species is associated with a disease, then an antibody that recognizes the test species can be used in a diagnostic test kit to determine the condition of a patient. The antibody is contacted with an appropriate sample from the patient to test for the presence of the test species, which is associated with a particular disease. The antibody can be incorporated into a diagnostic test kit that recognizes an epitope on a disease associated substance.

Where the test species is a population of malignant cells from a patient, e.g., cancer cells, then an antibody that recognizes the malignant cells while not recognizing healthy cells from the patient can be used to target drugs to the malignant cells. A sample of malignant cells is contacted with an AIM and antibodies that bind to the malignant cells are identified. A sample of healthy cells from the patient is contacted with a replica of the matrix, and antibodies that bind to the malignant cells, but not to the healthy cells, are selected. A hybridoma line producing selected antibodies is cultured to provide a source of the selected antibodies. A drug, e.g., cytotoxic agent, is then linked to the selected antibodies, and an effective amount of the drug-linked antibodies is administered to the patient.

### Other Embodiments

Other embodiments are within the following claims.

For example, it is not necessary that the matrix be constructed by immobilizing the antibodies or the amino acid sequences on a substrate. Each clone producing an antibody can be cultured separately, and each clone producing a peptide sequence can be cultured separately. Each antibody is tested individually with each peptide sequence. Correspondence between individual antibodies and the peptide sequences recognized by them can be recorded. A test species can then be tested against each of the individual antibody producing cultures. Any antibodies that bind to the test species can be identified,. and the specific peptide sequence recognized by the antibody can be determined by the corresponding peptide sequence-producing culture. Similarly, a test antibody can be tested against each of the individual peptide sequence producing cultures. The specific peptide sequence or sequences recognized by the test antibody can be determined directly by characterizing the unique peptide sequence produced by any cultures that show a positive binding response with the test antibody. This general method can readily be applied to any of the specific uses of a matrix set forth above.

The preferred embodiments of the invention are summarized in the following list:
a) A population of oligonucleotides, wherein:
   each member of the population consists essentially of between 1 and about 50 tandem sequences of from about 4 to about 12 nucleotide triplets,
   each member of the population has the same number of tandem repeating sequences of the same length,
   each of the tandem sequences encodes for a corresponding peptide sequence of about 4 to about 12 L-amino acid residues, and
   the population encodes for at least about 10% of all peptide sequences of the selected length.
aa) The oligonucleotide population of item a) wherein each member of the population comprises a single copy of the sequence of nucleotide triplets.
ab) The oligonucleotide population of item a) wherein each sequence comprises from 5 to 7 nucleotide triplets.
ac) The oligonucleotide population of item aa) wherein the population is generated by shearing of mammalian genetic material.
ad) The oligonucleotide population of item a) wherein the population is chemically synthesized from the component nucleic acids.
b) A process for the preparation of a population of peptides coded for by the oligonucleotides of item a) wherein:
   each member of the population comprises from 1 to about 50 tandem peptide sequences of about 4 to about 12 4 L-amino acid residues,
   each member of the population has the same number of tandem sequences of the same length, and
   the population contains at least about 10% of all possible peptide sequences of the selected length,
   which process comprises
   the random shearing of proteins or the random chemical synthesis from the component L-amino acids,
   or preparing the corresponding oligonucleotides by the random cleavage of biological genetic material or the random synthesis from the component nucleic acids, inserting the nucleotide sequences into vectors, transferring the vectors into appropriate host cells, culturing the cells and recovering the peptides.
ba) The process of item b) wherein each member of the population comprises a single copy of the peptide sequence.
bb) The process of item b) wherein each sequence comprises from 5 to 7 L-amino acid residues.
bc) The process of item ba) wherein the population is generated by shearing of proteins.
bd) The process of item b) wherein the population is chemically synthesized from the component L-amino acids.
c) A population of vectors, wherein each member of the population comprises a substantially identical autonomously replicating nucleic acid sequence wherein at least a portion of each sequence is a structural gene, and an
   oligonucleotide insert as defined in claim 1 recombinantly inserted into said structural gene within said vector, a significant proportion of the vector population being capable of expressing their recombinant structural genes when transferred into appropriate host cells, and wherein expression of the recombinant structural genes yields recombinant polypeptides comprising amino acid sequences as defined in claim 6.
be) The recombinant polypeptide population produced by the vector population of item c).
bf) The recombinant polypeptide population of item be), wherein each tandem unit of nucleotide triplets comprises from 5 to 7 nucleotide triplets.
cc) The vector population of item c) wherein the replicating sequence is a plasmid.
cd) The vector population of item cc) wherein the plasmid is pBR322.
ce) The vector population of item c) wherein the replicating sequence is a virus.
cf) The vector population of item ce) wherein the virus is lambda-gt 11.
cg) The vector population of item ce), wherein the virus is a strain of vaccinia.
ch) The vector population of item c) wherein the replicating sequence comprises a filamentous bacteriophage.
ci) The vector population of item ch) wherein the filamentous bacteriophage is pUC8.
e) A heterogeneous population of antibodies, comprising antibodies capable of binding to substantially every member of the peptide population of item b)
ea) A method of producing a heterogeneous population of antibodies, the method comprising the steps of:
   harvesting lymph cells from a mammal that has not been antigenically stimulated with a particular antigen,
   fusing the lymph cells with myeloma cells to produce hybridoma cells, and
   culturing individual hybridoma cell lines, the cell lines capable of producing antigens that are capable of recognizing a broad range of antigens.
eb) The method of item ea), wherein the mammal is raised aseptically until the lymph cells are harvested.
ec) The method of item ea), wherein the lymph cells are harvested from a fetal mammal or from a neonatal mammal not yet capable of responding to antigenic stimulation.
f) A population of binding pairs comprising:
   a population of peptide sequences of the same length, the length being about 4 to about 12 L-amino acid residues, the population comprising at least 10% of all peptide sequences of the selected length, and
   a heterogeneous population of antibodies comprising antibodies capable of binding to substantially every member of the oligopeptide population,
   substantially every member of the peptide population being bounded to its corresponding antibody.
g) A matrix comprising a substrate on which the sequences can be immobilized and a population of peptides sequences of the same length as defined in item b), the length being about 4 to about 12 L-amino acid residues, the population comprising at least 10% of all peptide sequences of the selected length, and
   a heterogeneous population of antibodies comprising antibodies capable of binding to substantially every member of the oligopeptide population.
ga) The matrix of item g), wherein each of the peptide sequences is immobilized on an appropriate substrate and the immobilized peptide sequences are contacted with the antibodies.
gb) The matrix of item ga), wherein each of the antibodies is labeled with an appropriate label that does not interfere substantially with binding and provides a means for identifying binding pairs.
gc) The matrix of item g) wherein each of the antibodies is immobilized on an appropriate substrate and the immobilized antibodies are contacted with the peptide sequences.
gd) The matrix of item gc), wherein each of the peptide sequences is labeled with an appropriate label that does not interfere substantially with binding and provides a means for identifying binding pairs.
ge) The matrix of item gd) wherein each of the peptide sequences is located on the surface of a fusion protein or modified vector, the protein or vector itself comprising the label.
gf) The matrix of item g, wherein each of the peptide sequences is contacted with each of the antibodies until at least one peptide sequence-antibody binding pair is identified.
i) A method for constructing a matrix according to item g) comprising:
   obtaining a population of peptide sequences having about 4 to about 12 L-amino acid residues, each member of the population having the same length, the population comprising at least 10% of all peptide sequences of the predetermined length,
   obtaining a heterogeneous population of antibodies, comprising antibodies capable of binding to substantially every member of the peptide sequence population, and
   contacting the antibodies with the peptide sequences for a sufficient amount of time and under appropriate conditions so that at least one peptide sequence-antibody binding pair is created.
ia) The method of item i), further comprising the step of:
   labeling the antibodies, the peptide sequences, or both with an appropriate label that does not interfere substantially with binding and provides a means for identifying any binding pairs.
ib) The method of item i), wherein each of the peptide sequences is purified, each of the antibodies is purified, and each of the peptide sequences is contacted with each of the antibodies until at least one peptide sequence-antibody binding pair is identified.
ic) The method of item ib), wherein each of the peptide sequences is contacted individually with each of the antibodies until at least one peptide sequence-antibody binding pair is identified.
id) The method of item i), wherein each of the peptide sequences is immobilized on an appropriate substrate and the immobilized peptide sequences are contacted with the antibodies.
ie) The method of item id), wherein each of the antibodies is labeled with an appropriate label that does not interfere substantially with binding and provides a means for identifying binding pairs.
if) The method of item i), wherein each of the antibodies is immobilized on an appropriate substrate and the immobilized antibodies are contacted with the peptide sequences.
ig) The method of item if), wherein each of the peptide sequences is labeled with an appropriate label that does not interfere substantially with binding but provides a means for identifying binding pairs.
ih) The method of item ig) wherein each of the peptide sequences is located on the surface of a fusion protein or modified vector, the protein or vector itself comprising the label.
ii) The method of item i) wherein each of the peptide sequences is translated from a genetically engineered vector as a portion of a larger fusion polypeptide.
ij) The method of item ii) wherein each peptide sequence is excised from its parent polypeptide.
j) A method for determining immunological and/or genotypic properties of a test species, wherein the test species is an antibody, virus, bacteriophage, enzyme, protein, polypeptide, non-peptide drug, or non-peptide bioactive substance, the method comprising the steps of:
   constructing a matrix according to item g) comprising a population of peptide sequences of the same length, the length being about 4 to about 12 L-amino acid residues, the population comprising at least 10% of all peptide sequences of the selected length; and a heterogeneous population of antibodies comprising antibodies capable of binding to substantially every member of the peptide sequence population;
   contacting the antibodies with the peptide sequences, for a sufficient amount of time and under appropriate conditions so that at least one peptide sequence-antibody binding pair is created,
   contacting the test species with the matrix,
   observing where the test species disturbs the binding pairs, and identifying the peptide sequence or the antibody at the site or sites where binding is disturbed.
k) A method of identifying a specific peptide sequence that has sufficient conformational similarity to an antibody recognition site on a test species that an antibody capable of recognizing and binding to the recognition site may also be capable of binding to the peptide sequence, the method comprising the steps of:
   contacting the test species with a matrix according to item g),
   observing where the test species disturbs the binding pairs, and
   identifying a peptide sequence comprising a binding pair disturbed by the presence of the test species.
ka) The method of item k), wherein the matrix is a peptide immobilized matrix wherein each immobilized peptide sequence forms a binding pair with a corresponding antibody, and the peptide sequence immobilized at a site where binding is disturbed is identified.
kb) The method of item k), wherein the matrix is an antibody immobilized matrix wherein each immobilized antibody forms a binding pair with a corresponding peptide sequence, and a peptide sequence displaced by the presence of the test species is identified.
m) A method of characterizing a recombinant gene product of a gene expression library, the method comprising the steps of:
   contacting the recombinant gene product with a matrix,
   observing where the gene product disturbs the binding pairs, and
   identifying the peptide sequence comprising a binding pair disturbed by the presence of the recombinant gene product.
ma) The method of item m), wherein the matrix is a peptide immobilized matrix wherein each immobilized peptide sequence forms a binding pair with a corresponding antibody, and the peptide sequence immobilized at a site where binding is disturbed is identified.
mb) The method of item m), wherein the matrix is an antibody immobilized matrix wnerein each immobilized antibody forms a binding pair with a corresponding peptide sequence, and a peptide sequence displaced by the presence of the recombinant gene product is identified.
n) A method of locating, in a genome, the gene encoding for a protein, enzyme, or peptide, the method comprising:
   contacting the protein, enzyme, or peptide with a matrix,
   observing where the protein distrubs the binding pairs,
   identifying the recombinant cell line that produced a peptide sequence comprising a binding pair disturbed by the presence of the protein, enzyme, or peptide, and
   using the nucleotide sequence of the oligonucleotide insert encoding for the peptide sequence as a DNA probe to locate the gene encoding for the protein.
na) The method of item n), wherein the matrix is a peptide immobilized matrix wherein each immobilized peptide sequence forms a binding pair with a corresponding antibody, and the peptide sequence immobilized at a site where binding is disturbed is identified.
nb) The method of item n, wnerein the matrix is an antibody immobilized matrix wnerein each immobilized antibody forms a binding pair with a corresponding peptide sequence, and a peptide sequence displaced by the presence of the protein, enzyme, or peptide is identified.
o) A method of determining a peptide sequence recognized by a first antibody, the method comprising the steps of:
   contacting the first antibody with a matrix according to item g),
   observing where the first antibody binds to a matrix-associated peptide sequence, and
   identifying the peptide sequence,
oa) The method of item o), wherein the matrix is a peptide immobilized matrix and the peptide sequence immobilized at a site where binding is disturbed is identified.
ob) The method of item o), wherein the matrix is an antibody immobilized matrix wherein each immobilized antibody forms a binding pair with a corresponding peptide sequence, and a peptide displaced by the presence of the first antibody is identified.
p) A method of determining the nucleotide sequence that encodes for a peptide sequence recognized by a first antibody, the method comprising the steps of:
   contacting the first antibody with a matrix according to item g),
   observing where the first antibody binds to a matrix-associated peptide sequence,
   identifying the genetically recombinant cell line that produced the peptide sequence, and
   determining the sequence of the oligonucleotide encoding for the peptide sequence inserted in the vector transferred into the cell line.
pa) The method of item p), wherein the matrix is a peptide immobilized matrix and the peptide sequence immobilized at a site where binding is disturbed is identified.
pb) The method of item p), wherein the matrix is an antibody immobilized matrix wherein each immobilized antibody forms a binding pair with a corresponding peptide sequence, and a peptide sequence displaced by the presence of the first antibody is identified.

## Claims

1. A population of oligonucleotides, wherein:
each member of the population consists essentially of between 1 and about 50 tandem sequences of from about 4 to about 12 nucleotide triplets,
each member of the population has the same number of tandem repeating sequences of the same length,
each of the tandem sequences encodes for a corresponding peptide sequence of about 4 to about 12 L-amino acid residues, and
the population encodes for at least about 10 % of all peptide sequences of the selected length.

2. The oligonucleotide population of claim 1 wherein each member of the population comprises a single copy of the sequence of nucleotide triplets.

3. The oligonucleotide population of claim 1 wherein each sequence comprises from 5 to 7 nucleotide triplets.

4. The oligonucleotide population of claim 2 wherein the population is generated by shearing of mammalian genetic material.

5. The oligonucleotide population of claim 1 wherein the population is chemically synthesized from the component nucleic acids.

6. A process for the preparation of a population of peptides coded for by the oligonucleotides of claim 1 wherein:
each member of the population comprises from 1 to about 50 tandem peptide sequences of about 4 to about 12 L-amino acid residues,
each member of the population has the same number of tandem sequences of the same length, and
the population contains at least about 10% of all possible peptide sequences of the selected length,
which process comprises
the random shearing of proteins or the random chemical synthesis from the component L-amino acids,
or preparing the corresponding oligonucleotides by the random cleavage of biological genetic material or the random synthesis from the component nucleic acids, inserting the nucleotide sequences into vectors, transferring the vectors into appropriate host cells, culturing the cells and recovering the peptides.

7. The process of claim 6 wherein each member of the population comprises a single copy of the peptide sequence.

8. The process of claim 6 wherein each sequence comprises from 5 to 7 L-amino acid residues.

9. The process of claim 7 wherein the population is generated by shearing of proteins.

10. The process of claim 6 wherein the population is chemically synthesized from the component L-amino acids.

11. A population of vectors, wherein each member of the population comprises a substantially identical autonomously replicating nucleic acid sequence wherein at least a portion of each sequence is a structural gene, and an
oligonucleotide insert as defined in claim 1 recombinantly inserted into said structural gene within said vector, a significant proportion of the vector population being capable of expressing their recombinant structural genes when transferred into appropriate host cells, and wherein expression of the recombinant structural genes yields recombinant polypeptides comprising amino acid sequences as defined in claim 6.

12. The recombinant polypeptide population produced by the vector population of claim 11.

13. The recombinant polypeptide population of claim 12, wherein each tandem unit of nucleotide triplets comprises from 5 to 7 nucleotide triplets.

14. A matrix comprising a substrate on which the sequences can be immobilized and a population of peptides sequences of the same length as defined in claim 6, the length being about 4 to about 12 L-amino acid residues, the population comprising at least 10% of all peptide sequences of the selected length, and
a heterogeneous population of antibodies comprising antibodies capable of binding to substantially every member of the oligopeptide population.

15. A method for constructing a matrix according to claim 14 comprising:
obtaining a population of peptide sequences having about 4 to about 12 L-amino acid residues, each member of the population having the same length, the population comprising at least 10% of all peptide sequences of the predetermined length,
obtaining a heterogeneous population of antibodies, comprising antibodies capable of binding to substantially every member of the peptide sequence population, and
contacting the antibodies with the peptide sequences for a sufficient amount of time and under appropriate conditions so that at least one peptide sequence-antibody binding pair is created.

16. The method of claim 15, further comprising the step of:
labeling the antibodies, the peptide sequences, or both with an appropriate label that does not interfere substantially with binding and provides a means for identifying any binding pairs.

17. The method of claim 15, wherein each of the peptide sequences is purified, each of the antibodies is purified, and each of the peptide sequences is contacted with each of the antibodies until at least one peptide sequence-antibody binding pair is identified.

18. The method of claim 17, wherein each of the peptide sequences is contacted individually with each of the antibodies until at least one peptide sequence-antibody binding pair is identified.

19. The method of claim 15, wherein each of the peptide sequences is immobilized on an appropriate substrate and the immobilized peptide sequences are contacted with the antibodies.

20. The method of claim 17, wherein each of the antibodies is labeled with an appropriate label that does not interfere substantially with binding and provides a means for identifying binding pairs.

21. The method of claim 15 wherein each of the peptide sequences is translated from a genetically engineered vector as a portion of a larger fusion polypeptide.

22. A method for determining immunological and/or genotypic properties of a test species, wherein the test species is an antibody, virus, bacteriophage, enzyme, protein, polypeptide, non-peptide drug, or non-peptide bioactive substance, the method comprising the steps of:
constructing a matrix according to claim 14 comprising a population of peptide sequences of the same length, the length being about 4 to about 12 L-amino acid residues, the population comprising at least 10% of all peptide sequences of the selected length; and a heterogeneous population of antibodies comprising antibodies capable of binding to substantially every member of the peptide sequence population;
contacting the antibodies with the peptide sequences, for a sufficient amount of time and under appropriate conditions so that at least one peptide sequence-antibody binding pair is created,
contacting the test species with the matrix,
observing where the test species disturbs the binding pairs, and identifying the peptide sequence or the antibody at the site or sites where binding is disturbed.

23. A method of identifying a specific peptide sequence that has sufficient conformational similarity to an antibody recognition site on a test species that an antibody capable fo recognizing and binding to the recognition site may also be capable of binding to the peptide sequence, the method comprising the steps of:
contacting the test species with a matrix according to claim 14,
observing where the test species disturbs the binding pairs, and
identifying a peptide sequence comprising a binding pair disturbed by the presence of the test species.

24. The method of claim 23, wherein the matrix is a peptide immobilized matrix wherein each immobilized peptide sequence forms a binding pair with a corresponding antibody, and the peptide sequence immobilized at a site where binding is disturbed is identified.

25. The method of claim 23, wherein the matrix is an antibody immobilized matrix wherein each immobilized antibody forms a binding pair with a corresponding peptide sequence, and a peptide sequence displaced by the presence of the test species is identified.

26. A method of locating, in a genome, the gene encoding for a protein, enzyme, or peptide, the method comprising:
contacting the protein, enzyme, or peptide with a matrix according to claim 14,
observing where the protein disturbs the binding pairs,
identifying the recombinant cell line comprising a vector of claim 11 which cell line produced a peptide sequence comprised in a binding pair disturbed by the presence of the protein, enzyme, or peptide, and
using the nucleotide sequence of the oligonucleotide sequence of the oligonucleotide insert encoding for the peptide sequence as a DNA probe to locate the gene encoding for the protein.

27. A method of determining a peptide sequence recognized by a first antibody, the method comprising the steps of:
contacting the first antibody with a matrix according to claim 14,
observing where the first antibody binds to a matrix-associated peptide sequence, and
identifying the peptide sequence.

28. The method of claim 27, wherein the matrix is a peptide immobilized matrix and the peptide sequence immobilized at a site where binding is disturbed is identified.

29. The method of claim 27, wherein the matrix is an antibody immobilized matrix wherein each immobilized antibody forms a binding pair with a corresponding peptide sequence, and a peptide displaced by the presence of the first antibody is identified.

## Patentansprüche

1. Population von Oligonucleotiden, wobei:
jedes Mitglied der Population im wesentlichen aus 1 bis etwa 50 Tandemsequenzen mit etwa 4 bis etwa 12 Nucleotidtripletts besteht,
jedes Mitglied der Population die gleiche Anzahl von Tandem-Wiederholungssequenzen der gleichen Länge aufweist,
jede der Tandemsequenzen eine entsprechende Peptidsequenz mit etwa 4 bis etwa 12 L-Aminosäureresten codiert, und
die Population mindestens etwa 10 % aller Peptidsequenzen der ausgewählten Länge codiert.

2. Oligonucleotidpopulation nach Anspruch 1, wobei jedes Mitglied der Population eine Einzelkopie der Nucleotidtriplettsequenz umfaßt.

3. Oligonucleotidpopulation nach Anspruch 1, wobei jede Sequenz 5 bis 7 Nucleotidtripletts umfaßt.

4. Oligonucleotidpopulation nach Anspruch 2, wobei die Population durch Scheren von genetischem Material von Säugern erzeugt wird.

5. Oligonucleotidpopulation nach Anspruch 1, wobei die Population aus den Nucleinsäurebestandteilen chemisch synthetisiert wird.

6. Verfahren zur Herstellung einer Population von Peptiden, die durch die Oligonucleotide nach Anspruch 1 codiert werden, wobei:
jedes Mitglied der Population 1 bis etwa 50 Tandem-Peptidsequenzen mit etwa 4 bis etwa 12 L-Aminosäureresten umfaßt,
jedes Mitglied der Population die gleiche Anzahl von Tandemsequenzen der gleichen Länge aufweist, und
die Population mindestens etwa 10 % aller möglichen Peptidsequenzen der ausgewählten Länge enthält,
wobei das Verfahren aie folgenden Schritte umfaßt:
das zufällige Scheren von Proteinen oder die zufällige chemische Synthese aus den L-Aminosäurebestandteilen,
oder die Herstellung der entsprechenden Oligonucleotide durch zufällige Spaltung von biologischem genetischem Material oder die zufällige Synthese aus den Nucleinsäurebestandteilen, Insertion der Nucleotidsequenzen in Vektoren, Transfer der Vektoren in geeignete Wirtszellen, Züchtung der Zellen und Gewinnung der Peptide.

7. Verfahren nach Anspruch 6, wobei jedes Mitglied der Population eine Einzelkopie der Peptidsequenz umfaßt.

8. Verfahren nach Anspruch 6, wobei jede Sequenz 5 bis 7 L-Aminosäurereste umfaßt.

9. Verfahren nach Anspruch 7, wobei die Population durch Scheren von Proteinen erzeugt wird.

10. Verfahren nach Anspruch 6, wobei die Population aus den L-Aminosäurebestandteilen chemisch synthetisiert wird.

11. Population von Vektoren, wobei jedes Mitglied der Population eine im wesentlichen identische autonom replizierbare Nucleinsäuresequenz umfaßt, wobei mindestens ein Teil jeder Sequenz ein Strukturgen ist, und
eine Oligonucleotidinsertion gemäß der Definition in Anspruch 1, die rekombinant in das Strukturgen innerhalb des Vektors insertiert ist, wobei ein signifikanter Anteil der Vektorpopulation deren rekombinante Strukturgene exprimieren kann, wenn sie in die entsprechenden Wirtszellen transferiert werden, und wobei die Expression der rekombinanten Strukturgene rekombinante Polypeptide erzeugt, die Aminosäuresequenzen gemäß der Definition in Anspruch 6 umfassen.

12. Rekombinante Polypeptidpopulation, produziert von der Vektorpopulation nach Anspruch 11.

13. Rekombinante Polypeptidpopulation nach Anspruch 12, wobei jede Tandemeinheit von Nucleotidtripletts 5 bis 7 Nucleotidtripletts umfaßt.

14. Matrix, umfassend ein Substrat, an dem die Sequenzen immobilisiert werden können, und eine Population von Peptidsequenzen der gleichen Länge gemäß der Definition in Anspruch 6, wobei die Länge bei etwa 4 bis etwa 12 L-Aminosäureresten liegt, wobei die Population mindestens 10 % aller Peptidsequenzen der ausgewählten Länge umfaßt, und
eine heterogene Population von Antikörpern, umfassend Antikörper, die im wesentlichen an jedes Mitglied der Oligopeptidpopulation binden können.

15. Verfahren zur Konstruktion einer Matrix nach Anspruch 14, umfassend:
Gewinnung einer Population von Peptidsequenzen mit etwa 4 bis etwa 12 L-Aminosäureresten, wobei jedes Mitglied der Population die gleiche Länge hat, wobei die Population mindestens 10 % aller Peptidsequenzen der vorbestimmten Länge umfaßt,
Gewinnung einer heterogenen Population von Antikörpern, umfassend Antikörper, die im wesentlichen an jedes Mitglied der Peptidsequenzpopulation binden können, und
Inkontaktbringen der Antikörper mit den Peptidsequenzen für einen ausreichenden Zeitraum und unter geeigneten Bedingungen, so daß mindestens ein Peptidsequenz-Antikörper-Bindungspaar erzeugt wird.

16. Verfahren nach Anspruch 15, weiterhin umfassend den folgenden Schritt:
Markieren der Antikörper, der Peptidsequenzen oder beider mit einem geeigneten Marker, der die Bindung nicht wesentlich stört und ein Mittel zur Identifizierung von Bindungspaaren liefert.

17. Verfahren nach Anspruch 15, wobei jede der Peptidsequenzen gereinigt wird, jeder der Antikörper gereinigt wird und jede der Peptidsequenzen mit jedem der Antikörper in Kontakt gebracht wird, bis mindestens ein Peptidsequenz-Antikörper-Bindungspaar identifiziert ist.

18. Verfahren nach Anspruch 17, wobei jede der Peptidsequenzen individuell mit jedem der Antikörper in Kontakt gebracht wird, bis mindestens ein Peptidsequenz-Antikörper-Bindungspaar identifiziert ist.

19. Verfahren nach Anspruch 15, wobei jede der Peptidsequenzen an einem geeigneten Substrat immobilisiert ist und die immobilisierten Peptidsequenzen mit den Antikörpern in Kontakt gebracht werden.

20. Verfahren nach Anspruch 17, wobei jeder der Antikörper mit einem geeigneten Marker markiert wird, der die Bindung nicht wesentlich stört und ein Mittel zur Identifizierung von Bindungspaaren liefert.

21. Verfahren nach Anspruch 15, wobei jede der Peptidsequenzen von einem gentechnisch hergestellten Vektor als Teil eines größeren Fusionspolypeptids translatiert wird.

22. Verfahren zur Bestimmung von immunologischen und/oder genotypischen Eigenschaften einer Testspezies, wobei die Testspezies ein Antikörper, Virus, Bakteriophage, Enzym, Protein, Polypeptid, nicht-peptidischer Wirkstoff oder eine nicht-peptidische bioaktive Substanz ist, wobei das Verfahren die folgenden Schritte umfaßt:
Konstruktion einer Matrix nach Anspruch 14, umfassend eine Population von Peptidsequenzen der gleichen Länge, wobei die Länge bei etwa 4 bis etwa 12 L-Aminosäureresten liegt, wobei die Population mindestens 10 % aller Peptidsequenzen der ausgewählten Länge umfaßt; und eine heterogene Population von Antikörpern, umfassend Antikörper, die im wesentlichen an jedes Mitglied der Peptidsequenzpopulation binden können,
Inkontaktbringen der Antikörper mit den Peptidsequenzen für einen ausreichenden Zeitraum und unter geeigneten Bedingungen, so daß mindestens ein Peptidsequenz-Antikörper-Bindungspaar erzeugt wird,
Inkontaktbringen der Testspezies mit der Matrix,
Beobachten, wo die Testspezies die Bindungspaare stört, und Identifizierung der Peptidsequenz oder des Antikörpers an der Stelle oder den Stellen, wo die Bindung gestört ist.

23. Verfahren zur Identifizierung einer spezifischen Peptidsequenz, die eine ausreichende Konformationsähnlichkeit mit einer Antikörpererkennungsstelle auf einer Testspezies hat, so daß ein Antikörper, der die Erkennungsstelle erkennen und daran binden kann, auch an die Peptidsequenz binden kann, wobei das Verfahren die folgenden Schritte umfaßt:
Inkontaktbringen der Testspezies mit einer Matrix nach Anspruch 14,
Beobachten, wo die Testspezies die Bindungspaare stört, und
Identifizierung einer Peptidsequenz, die ein Bindungspaar umfaßt, das durch die Gegenwart der Testspezies gestört wird.

24. Verfahren nach Anspruch 23, wobei die Matrix eine Matrix mit immobilisiertem Peptid ist, wobei jede immobilisierte Peptidsequenz ein Bindungspaar mit einem entsprechenden Antikörper erzeugt, und die Peptidsequenz, die an einer Stelle immobilisiert ist, wo die Bindung gestört wird, identifiziert wird.

25. Verfahren nach Anspruch 23, wobei die Matrix eine Matrix mit immobilisiertem Antikörper ist, wobei jeder immobilisierte Antikörper ein Bindungspaar mit einer entsprechenden Peptidsequenz erzeugt, und eine Peptidsequenz, die durch die Gegenwart der Testspezies ersetzt wird, identifiziert wird.

26. Verfahren zur Lokalisierung des Gens in einem Genom, das ein Protein, Enzym oder Peptid codiert, wobei das Verfahren folgende Schritte umfaßt:
Inkontaktbringen des Proteins, Enzyms oder Peptids mit einer Matrix nach Anspruch 14,
Beobachten, wo das Protein die Bindungspaare stört,
Identifizierung der rekombinanten Zellinie, die einen Vektor nach Anspruch 11 umfaßt, wobei die Zellinie eine Peptidsequenz produziert, die in einem Bindungspaar vorliegt, das durch die Gegenwart des Proteins, Enzyms oder Peptids gestört wird, und
Verwendung der Nucleotidsequenz der Oligonucleotidsequenz der Oligonucleotidinsertion, die die Peptidsequenz codiert, als eine DNA-Sonde zur Lokalisierung des Gens, das das Protein codiert.

27. Verfahren zur Bestimmung einer Peptidsequenz, die durch einen ersten Antikörper erkannt wird, wobei das Verfahren die folgenden Schritte umfaßt:
Inkontaktbringen des ersten Antikörpers mit einer Matrix nach Anspruch 14,
Beobachten, wo der erste Antikörper an eine Matrix-assoziierte Peptidsequenz bindet, und
Identifizierung der Peptidsequenz.

28. Verfahren nach Anspruch 27, wobei die Matrix eine Matrix mit immobilisiertem Peptid ist und die Peptidsequenz, die an einer Stelle immobilisiert ist, wo die Bindung gestört wird, identifiziert wird.

29. Verfahren nach Anspruch 27, wobei die Matrix eine Matrix mit immobilisiertem Antikörper ist, wobei jeder immobilisierte Antikörper ein Bindungspaar mit einer entsprechenden Peptidsequenz erzeugt, und ein Peptid, das durch die Gegenwart des ersten Antikörpers ersetzt wird, identifiziert wird.

## Revendications

1. Population d'oligonucléotides, dans laquelle :
chaque élément de la population est constitué essentiellement de 1 à environ 50 séquences en tandem, ayant environ 4 à environ 12 triplets nucléotidiques,
chaque élément de la population possède le même nombre de séquences répétées en tandem, de même longueur,
chacune des séquences en tandem code pour une séquence peptidique correspondante ayant d'environ 4 à environ 12 résidus d'acides L-aminés, et
la population code pour au moins environ 10 % de l'ensemble des séquences peptidiques ayant la longueur sélectionnée.

2. Population d'oligonucléotides selon la revendication 1, dans laquelle chaque élément de la population comprend une copie unique de la séquence des triplets nucléotidiques.

3. Population d'oligonucléotides selon la revendication 1, dans laquelle chaque séquence comprend de 5 à 7 triplets nucléotidiques.

4. Population d'oligonucléotides selon la revendication 2, dans laquelle la population est générée par cisaillement d'un matériel génétique de mammifère.

5. Population d'oligonucléotides selon la revendication 1, dans laquelle la population est synthétisée par voie chimique à partir des acides nucléiques constituants.

6. Procédé pour préparer une population de peptides codés par les oligonucléotides selon la revendication 1, dans lequel :
chaque élément de la population comprend de 1 à environ 50 séquences peptidiques en tandem, constituées d'environ 4 à environ 12 résidus d'acides L-aminés,
chaque élément de la population a le même nombre de séquences en tandem, avec la même longueur, et
la population contient au moins environ 10 % de l'ensemble des séquences peptidiques possibles ayant la longueur sélectionnée,
lequel procédé consiste :
à procéder au cisaillement aléatoire de protéines, ou à la synthèse chimique aléatoire à partir des acides L-aminés constituants,
ou à préparer les oligonucléotides correspondants par clivage aléatoire d'un matériel génétique biologique, ou par synthèse aléatoire à partir des acides nucléiques constituants, à insérer les séquences nucléotidiques dans des vecteurs, à transférer les vecteurs dans des cellules hôtes appropriées, à cultiver les cellules et à récupérer les peptides.

7. Procédé selon la revendication 6, dans lequel chaque élément de la population comprend une copie unique de la séquence peptidique.

8. Procédé selon la revendication 6, dans lequel chaque séquence comprend de 5 à 7 résidus d'acides L-aminés.

9. Procédé selon la revendication 7, dans lequel la population est générée par cisaillement de protéines.

10. Procédé selon la revendication 6, dans lequel la population est synthétisée par voie chimique à partir des acides L-aminés constituants.

11. Population de vecteurs, dans laquelle chaque élément de la population comprend une séquence d'acide nucléique à réplication autonome, essentiellement identique, où au moins une portion de chaque séquence est un gène de structure, et
un insert oligonucléotidique, tel que défini dans la revendication 1, inséré par une technique de recombinaison dans ledit gène de structure se trouvant à l'intérieur dudit vecteur, une proportion significative de la population de vecteurs étant capable d'exprimer leurs gènes de structure recombinants après transfert dans des cellules hôtes appropriées, et où l'expression des gènes de structure recombinants donne des polypeptides recombinants comprenant des séquences d'acides aminés tels que définies dans la revendication 6.

12. Population de polypeptides recombinants produits par la population de vecteurs selon la revendication 11.

13. Population de polypeptides recombinants selon la revendication 12, dans laquelle chaque unité en tandem de triplets nucléotidiques comprend de 5 à 7 triplets nucléotidiques.

14. Matrice comprenant un substrat sur lequel les séquences peuvent être immobilisées et une population de séquences peptidiques ayant la même longueur que celle qui est définie dans la revendication 6, la longueur étant d'environ 4 à environ 12 résidus d'acides L-aminés, la population représentant au moins 10 % de la totalité des séquences peptidiques ayant la longueur sélectionnée, et
une population hétérogène d'anticorps, comprenant des anticorps capables de se fixer pour ainsi dire à chaque élément de la population d'oligopeptides.

15. Procédé pour synthétiser une matrice selon la revendication 14, qui consiste :
à obtenir une population de séquences peptidiques ayant d'environ 4 à environ 12 résidus d'acides L-aminés, chaque élément de la population ayant la même longueur, la population représentant au moins 10 % de la totalité des séquences peptidiques ayant la longueur prédéterminée,
à obtenir une population hétérogène d'anticorps, comprenant des anticorps capables de se fixer pour ainsi dire à chaque élément de la population de séquences peptidiques, et
à mettre en contact les anticorps avec les séquences peptidiques pendant un laps de temps suffisant et dans des conditions appropriées pour créer au moins un couple de fixation séquence peptidique-anticorps.

16. Procédé selon la revendication 15, qui comprend en outre l'étape consistant :
à marquer les anticorps, les séquences peptidiques ou les deux, avec un marqueur approprié qui n'interfère pas exagérément avec la fixation et qui fournit un moyen d'identification de tous couples de fixation.

17. Procédé selon la revendication 15, dans lequel chacune des séquences peptides est purifiée, chacun des anticorps est purifié, et chacune des séquences peptidiques est mise en contact avec chacun des anticorps jusqu'à identification d'au moins un couple de fixation séquence peptidique-anticorps.

18. Procédé selon la revendication 17, dans lequel chacune des séquences peptidiques est à titre individuel mise en contact avec chacun des anticorps jusqu'à identification d'au moins un couple de fixation séquence peptidique-anticorps.

19. Procédé selon la revendication 15, dans lequel chacune des séquences peptidiques est immobilisée sur un support approprié, et les séquences peptidiques immobilisées sont mises en contact avec les anticorps.

20. Procédé selon la revendication 17, dans lequel chacun des anticorps est marqué avec un marqueur approprié qui n'interfère pas exagérément avec la fixation et fournit un moyen d'identification des couples de fixation.

21. Procédé selon la revendication 15, dans lequel chacune des séquences peptidiques est traduite à partir d'un vecteur obtenu par génie génétique, en tant que portion d'un plus gros polypeptide de fusion.

22. Procédé pour déterminer les propriétés immunologiques et/ou génotypiques d'une espèce d'essai, où l'espèce d'essai est un anticorps, un virus, un bactériophage, une enzyme, une protéine, un polypeptide, un médicament non-peptidique ou une substance bioactive non-peptidique, le procédé comprenant les étapes consistant :
à synthétiser une matrice selon la revendication 14, comprenant une population de séquences peptidiques de même longueur, la longueur étant d'environ 4 à environ 12 résidus d'acides L-aminés, la population représentant au moins 10 % de l'ensemble des séquences peptidiques ayant la longueur sélectionnée ; et une population hétérogène d'anticorps comprenant des anticorps capables de se fixer pour ainsi dire à chaque élément de la population de séquences peptidiques ;
à mettre en contact les anticorps avec les séquences peptidiques, pendant un laps de temps suffisant et dans des conditions appropriées de façon à créer au moins un couple de fixation séquence peptidique-anticorps,
à mettre en contact l'espèce d'essai avec la matrice,
à observer le point où l'espèce d'essai perturbe les couples de fixation, et à identifier la séquence peptidique ou l'anticorps sur le ou les sites de perturbation de la fixation.

23. Procédé d'identification d'une séquence peptidique spécifique, ayant une similitude de conformation vis-à-vis d'un site de reconnaissance d'anticorps se trouvant sur une espèce d'essai, suffisante pour qu'un anticorps capable de reconnaître le site de reconnaissance et de s'y fixer puisse aussi être capable de se fixer à la séquence peptidique, le procédé comprenant les étapes consistant :
à mettre en contact l'espèce d'essai avec une matrice selon la revendication 14,
à observer le point où l'espèce d'essai perturbe les couples de fixation, et
à identifier une séquence peptidique comprenant un couple de fixation perturbé par la présence de l'espèce d'essai.

24. Procédé selon la revendication 23, dans lequel la matrice est une matrice à peptides immobilisés, où chaque séquence peptidique immobilisée forme un couple de fixation avec un anticorps correspondant, et on identifie la séquence peptidique immobilisée sur un site où la fixation est perturbée.

25. Procédé selon la revendication 23, dans lequel la matrice est une matrice à anticorps immobilisés, où chaque anticorps immobilisé forme un couple de fixation avec une séquence peptidique correspondante, et on identifie une séquence peptidique déplacée par la présence de l'espèce d'essai.

26. Procédé de localisation, dans un génome, du gène codant pour une protéine, une enzyme ou un peptide, le procédé consistant :
à mettre en contact la protéine, l'enzyme ou le peptide avec une matrice selon la revendication 14,
à observer le point où la protéine perturbe les couples de fixation,
à identifier la lignée cellulaire recombinante comprenant un vecteur selon la revendication 11, laquelle lignée cellulaire produit une séquence peptidique faisant partie d'un couple de fixation perturbé par la présence de la protéine, de l'enzyme ou du peptide, et
à utiliser la séquence nucléotidique de la séquence d'oligonucléotides de l'insert oligonucléotidique codant pour la séquence peptidique, en tant que sonde à ADN, pour localiser le gène codant pour la protéine.

27. Procédé pour déterminer une séquence peptidique reconnue par un premier anticorps, le procédé comprenant les étapes consistant :
à mettre en contact le premier anticorps avec une matrice selon la revendication 14,
à observer le point où le premier anticorps se fixe à une séquence peptidique associée à la matrice, et
à identifier la séquence peptidique.

28. Procédé selon la revendication 27, dans lequel la matrice est une matrice à peptides immobilisés, et on identifie la séquence peptidique immobilisée sur un site où la fixation est perturbée.

29. Procédé selon la revendication 27, dans lequel la matrice est une matrice à anticorps immobilisés, où chaque anticorps immobilisé forme un couple de fixation avec une séquence peptidique correspondante, et on identifie un peptide déplacé par la présence du premier anticorps.
